(19) Europäisches Patentamt
European Patent Office
Office européen des brevets

(11) Veröffentlichungsnummer: **0 107 806**
**B1**

# (12) EUROPÄISCHE PATENTSCHRIFT

(45) Veröffentlichungstag der Patentschrift: **24.05.89**

(21) Anmeldenummer: **83109791.0**

(22) Anmeldetag: **30.09.83**

(51) Int. Cl.⁴: **C 07 D 311/72**, C 07 D 303/32, C 07 D 303/26, C 07 C 43/23, C 07 C 41/26

## (54) Verfahren zur Herstellung von optisch aktiven Hydrochinonderivaten sowie von d-alpha-Tocopherol.

(30) Priorität: **27.10.82 CH 6270/82**

(43) Veröffentlichungstag der Anmeldung: **09.05.84 Patentblatt 84/19**

(45) Bekanntmachung des Hinweises auf die Patenterteilung: **24.05.89 Patentblatt 89/21**

(84) Benannte Vertragsstaaten: **AT BE CH DE FR GB IT LI NL**

(56) Entgegenhaltungen:
**EP-A- 0 058 945**

**JOURNAL OF ORGANIC CHEMISTRY, Band 46, Nr. 12, 5. Juni 1981, Seiten 2445-2450, American Chemical Society, US, N. COHEN et al.: "Studies on the total synthesis of (2R,4'R,8'R)-alpha-tocopherol (vitamin E). Stereospecific cyclizations leading to optically active chromans"**

(73) Patentinhaber: **F. HOFFMANN-LA ROCHE & CO. Aktiengesellschaft, CH-4002 Basel (CH)**

(72) Erfinder: **Barner, Richard, Dr., In den Reben 97, CH-4108 Witterswil (CH)**
Erfinder: **Hübscher, Josef, Dr., Spausel 1080, CH-5703 Seon (CH)**

(74) Vertreter: **Urech, Peter, Dr. et al, Grenzacherstrasse 124 Postfach 3255, CH-4002 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft ein neues Verfahren zur Herstellung von Hydrochinonderivaten, welche als Zwischenprodukte für die Herstellung von d-α-Tocopherol (natürliches Vitamin E) geeignet sind, sowie ein Verfahren zur Herstellung von d-α-Tocopherol selbst. Die Erfindung betrifft ferner neue Ausgangsprodukte in diesem Verfahren.

Es sind bereits einige Verfahren zur Herstellung von natürlichem Vitamin E bekannt, welche jedoch technisch nur von begrenztem Interesse sind, so daß natürliches Vitamin E bis heute nahezu ausschließlich aus natürlichen Quellen extrahiert wird.

So ist insbesondere aus der EP-A-0 058 945 ein Verfahren zur Herstellung von d-α-Tocopherol, ausgehend von einem Phytolepoxyd bekannt, welches jedoch insgesamt neun Stufen bis zum Endprodukt benötigt. Diese Zählweise ist ersichtlich aus dem Reaktionsschema der Seiten 2/7, im Zusammenhang mit der Beschreibung, Seiten 11 bis 15 und Beispiel 5.

Es bestand somit ein Bedürfnis nach einem technisch realisierbaren Verfahren, gemäß welchem natürliches Vitamin E in guter Ausbeute und mit hoher optischer Reinheit erhalten werden kann. Dies ist nunmehr mittels des erfindungsgemäßen Verfahrens möglich. Dieses benötigt nur mehr 5, bzw. 6 Stufen ex Phytolepoxid, siehe unten die Stufen VII→V→I→II→III→IV.

Die Verkürzung des Synthesewegs wird insbesondere in der originellen Struktur von I bzw. deren Auswahl in der gegenständlichen Reaktionsfolge gesehen.

Dieses Verfahren ist dadurch gekennzeichnet, daß man eine Verbindung der allgemeinen Formel I

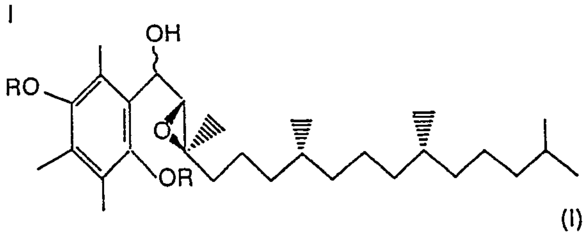

(I)

worin R eine übliche Ätherschutzgruppe darstellt, mittels eines komplexen Hydrides regioselektiv reduziert, daß man gewünschtenfalls eine so erhaltene Verbindung der allgemeinen Formel II

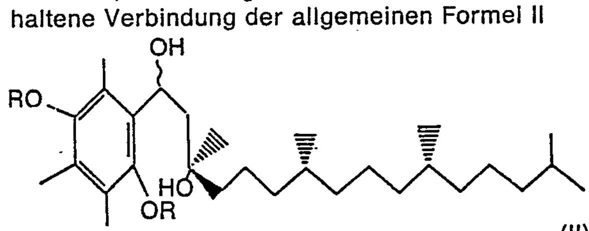

(II)

worin R die obige Bedeutung hat, in eine Verbindung der allgemeinen Formel III

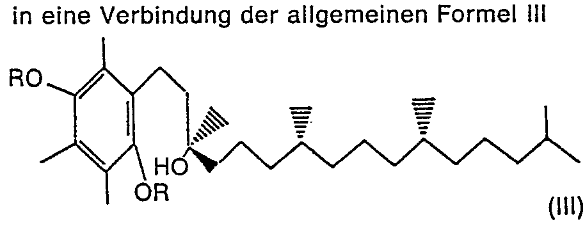

(III)

worin R die obige Bedeutung hat, überführt und diese, gewünschtenfalls, in d-α-Tocopherol der Formel IV

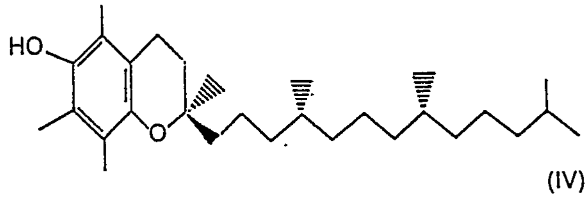

(IV)

überführt.

Der Ausdruck «Ätherschutzgruppe» bedeutet im Rahmen der Erfindung sowohl durch Hydrolyse abspaltbare Gruppen, wie etwa Trialkylsilylgruppen oder Alkoxyalkylgruppen, beispielsweise die Methoxymethylgruppe, oder auch die Tetrahydropyranylgruppe, als auch oxidativ abspaltbare Gruppen, wie etwa $C_1$–$C_6$ Alkyläthergruppen. Weiterhin bedeutet das Zeichen «◀», daß sich der entsprechende Rest oberhalb der Molekülebene befindet, während das Zeichen «//////» bedeutet, daß sich der entsprechende Rest unterhalb der Molekülebene befindet.

Die Verbindungen der allgemeinen Formel I sind neu und ebenfalls Gegenstand der Erfindung.

Die regioselektive Reduktion einer Verbindung der allgemeinen Formel I zu einer Verbindung der allgemeinen Formel II, d.h. die reduktive Spaltung des Epoxides, erfolgt zweckmäßig in einem inerten organischen Lösungsmittel. Als solche kommen insbesondere in Frage aliphatische und aromatische Kohlenwasserstoffe, wie z.B. Pentan, Hexan, Benzol oder Toluol, sowie Äther, wie Diäthyläther, tert. Butylmethyläther, Tetrahydrofuran, Dioxan, oder auch Gemische von Kohlenwasserstoffen und Äthern. Das Lösungsmittel bzw. das Lösungsmittelgemisch wird vorzugsweise so gewählt, daß das Ausgangsprodukt darin löslich ist.

Als Reduktionsmittel zur regioselektiven Öffnung des Epoxides kommen im Rahmen der Erfindung insbesondere komplexe Hydride in Frage. Als bevorzugte Beispiele von in Frage kommenden Hydriden werden genannt: Natrium bis(2-methoxy-äthoxy)aluminiumhydrid [Red-al®], Lithiumaluminiumhydrid, Lithiumborhydrid, Aluminiumborhydrid, Aluminiumhydrid oder Diboran. Ganz besonders bevorzugt sind hierbei Natrium bis(2-methoxy-äthoxy)aluminiumhydrid und Lithiumaluminiumhydrid.

Die Temperatur und der Druck sind keine kritischen Größen in diesem Zusammenhang, so daß die Reaktion ohne weiteres bei Raumtemperatur und Normaldruck durchgeführt werden kann.

Die Umsetzung einer Verbindung der allgemeinen Formel II zu einer Verbindung der allgemeinen Formel III kann in an sich bekannter Weise durchgeführt werden. Zweckmäßig erfolgt die Umsetzung durch Hydrierung in saurem Medium. Die Hydrierung kann in Gegenwart eines üblichen Hydrierkatalysators, z.B. Palladium oder Platin, mit oder ohne Trägermaterial erfolgen. Als Säuren kommen in Frage Mineralsäuren, wie Chlorwasserstoffsäure, Schwefelsäure oder auch Perchlorsäure, sowie Carbonsäuren, wie Ameisensäure

oder Essigsäure. Die Temperatur und der Druck sind bei dieser Umsetzung keine kritischen Größen, so daß diese ohne weiteres bei Raumtemperatur und Normaldruck durchgeführt werden kann.

Die Verbindungen der allgemeinen Formel III sind bekannt und können in bekannter Weise in d-α-Tocopherol übergeführt werden. Dies kann beispielsweise, falls der Rest R durch Hydrolyse abspaltbar ist, in einfacher Weise durch Behandlung mit einer Säure erfolgen. Falls der Rest R oxidativ abspaltbar ist, erfolgt die Überführung in einfacher Weise durch Behandlung mit z.B. Cerammoniumnitrat [$(Ce(NH_4)_2(NO_3)_6$] und anschließende reduktive Cyclisierung des so erhaltenen Chinons.

Die in dem erfindungsgemäßen Verfahren als Ausgangsmaterial verwendeten Verbindungen der allgemeinen Formel I sind neu. Sie können ausgehend von der Verbindung der Formel V

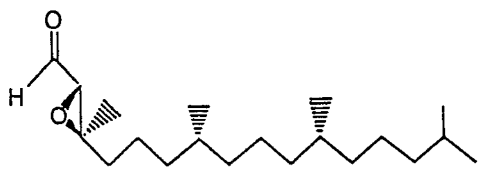

(V)

durch Umsetzung mit einer Verbindung der allgemeinen Formel VI

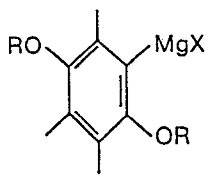

(VI)

worin R die obige Bedeutung hat und X ein Brom- oder Chloratom darstellt,
hergestellt werden.

Diese Umsetzung kann in an sich bekannter Weise, d.h. unter den für eine Grignard-Reaktion üblichen Bedingungen durchgeführt werden.

Zweckmäßig erfolgt dies in einem inerten organischen Lösungsmittel, beispielsweise in einem Äther, wie Diäthyläther oder Tetrahydrofuran und bei einer Temperatur von etwa −20°C bis etwa Raumtemperatur, vorzugsweise bei etwa −20°C bis etwa 0°C.

Die Verbindung der obigen Formel V ist ebenfalls neu und daher ebenfalls Gegenstand der Erfindung. Sie kann in einfacher Weise durch Oxidation der bekannten Verbindung der Formel VII

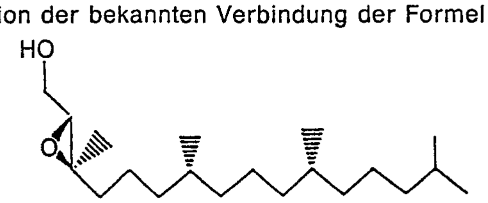

(VII)

hergestellt werden. Diese Oxidation kann in an sich bekannter Weise, beispielsweise mittels Pyridiniumchlorochromat in Methylenchlorid (Corey-Reagenz) erfolgen.

Die Verbindungen der Formel VI sind bekannte oder Analoge bekannter Verbindungen und können nach an sich bekannten Methoden hergestellt werden.

Beispiel 1

Eine Lösung von 342 mg (0,70 mMol) (1RS,2R,3R,7R,11R)-1-(2′,5′-Dimethoxy-3′,4′,6′-trimethylphenyl)-2,3-epoxi-3,7,11,15-tetramethylhexadecanol in 1 ml tert. Butylmethyläther wurde bei Raumtemperatur unter Rühren zu einer Suspension von 51 mg (2 mMol) Lithiumaluminiumhydrid in 10 ml tert. Butylmethyläther zugetropft und das Gemisch wurde anschließend noch 1 Stunde bei Raumtemperatur gerührt. Dann wurden 0,5 ml Wasser zugetropft, durch Natriumsulfat filtriert, mit Äther nachgewaschen und das Filtrat am Rotationsverdampfer eingeengt. Das so erhaltene Rohprodukt (341 mg) wurde an Kieselgel mit Toluol/Essigester (2:1) chromatographiert und man erhielt 284 mg (84%) (1RS,3R,7R,11R)-1-(2′,5′-Dimethoxy-3′,4′,6′-trimethylphenyl)-3,7,11,15-tetramethylhexadecan-1,3-diol, $[\alpha]_D^{20}$ = +0,2° (c = 2,16%, in Chloroform).

Das als Ausgangsmaterial verwendete (1RS,2R,3R,7R,11R)-1-(2′,5′-Dimethoxy-2′,4′,6′-trimethylphenyl)-2,3-epoxi-3,7,11,15-tetramethylhexadecanol wurde wie folgt hergestellt:

A) Zu einer Suspension von 1,6 g (5,45 mMol) Pyridiniumchlorochromat in 50 ml Methylenchlorid wurden unter Rühren bei Raumtemperatur 1,70 g (5,45 mMol) (2R,3R,7R,11R)-2,3-Epoxi-3,7,11,15-tetramethylhexadecanol in 5 ml Methylenchlorid zugetropft und das Gemisch wurde über Nacht bei Raumtemperatur weitergerührt. Dann wurde Hyflo zugegeben und mit 100 ml Äther 1 Stunde verrührt, anschließend filtriert und mit Äther nachgewaschen. Das Rohprodukt wurde an Kieselgel mit Toluol chromatographiert. Man erhielt 1,46 g (87%) (2R,3R,7R,11R)-2,3-Epoxi-3,7,11,15-tetramethylhexadecanal, $[\alpha]_D^{20}$ = −5,87° (c = 1,5%, in Chloroform).

B) 1 mMol 2,5-Dimethoxy-3,4,6-trimethylphenylmagnesiumbromid in 2 ml Tetrahydrofuran wurde bei 0°C unter Rühren zu einer Lösung von 300 mg (0,97 mMol) (2R,3R,7R,11R)-2,3-Epoxi-3,7,11,15-tetramethylhexadecanal in 10 ml Tetrahydrofuran zugetropft. Nach beendetem Zutropfen wurde noch 3 Stunden bei Raumtemperatur gerührt. Dann wurden 20 ml Wasser zugefügt und das Reaktionsgemisch 3mal mit je 20 ml Äther extrahiert. Die vereinigten organischen Phasen wurden über Natriumsulfat getrocknet und am Rotationsverdampfer eingeengt. Das so erhaltene Rohprodukt (502 mg) wurde mit Toluol/Essigester (2:1) an Kiesselgel chromatographiert und man erhielt 448 mg (94%) (1RS,2R,3R,7R,11R)-1-(2′,5′-Dimethoxy-3′,4′,6′-trimethylphenyl)-2,3-epoxi-3,7,11,15-tetramethylhexadecanol. $[\alpha]_D^{20}$ = −0,66° (c = 1,51%, in Chloroform).

Beispiel 2

Eine Lösung von 197 mg (0,40 mMol) (1RS,3S,7R,11R)-1-(2′,5′-Dimethoxy-3′,4′,6′-trimethylphenyl)-2,7,11,15-tetramethylhexadecan-1,3-

diol in 20 ml Methanol wurde nach Zugabe von 1 ml 70%iger Perchlorsäure in Gegenwart von 100 mg 10% Pd/C in einer Wasserstoffatmosphäre während 15 Stunden bei Raumtemperatur geschüttelt. Dann wurde mit Natriumcarbonat neutralisiert, nach Zugabe von 50 ml Essigester durch Hyflo filtriert und das Filtrat am Rotationsverdampfer eingeengt. Man erhielt 165 mg (85%) (3R,7R,11R)-1-(2',5'-Dimethoxy-3',4',6'-trimethylphenyl)-3,7,11,15-tetramethylhexadecan-3-ol. $[\alpha]^{20}_{D} = -0,67°$ (c = 0,90%, in Chloroform).

Beispiel 3

Zu einer Lösung von 530 mg (1,13 mMol) (3R,7R,11R)-1-(2',5'-Dimethoxy-3',4',6'-trimethylphenyl)-3,7,11,15-tetramethylhexadecan-3-ol in 50 ml Acetonitril wurden unter Rühren 1,38 g Cer(IV)-ammoniumnitrat in 5 ml Wasser zugegeben und diese Mischung wurde während 1 Stunde bei Raumtemperatur gerührt. Das Reaktionsgemisch wurde 3mal mit je 20 ml Methylenchlorid extrahiert, die vereinigten organischen Phasen wurden über Natriumsulfat getrocknet und am Rotationsverdampfer eingedampft. Man erhielt 480 mg (3'R,7'R,11'R)-2-(3'-Hydroxy-3',7',11',15'-tetramethylhexadecan-1'-yl)-3,4,5-trimethyl-1,4-benzochinon.

Das Produkt wurde in 100 ml Methanol gelöst und über Pd/C-10% hydriert. Dann wurden 0,5 ml konzentrierte Salzsäure zugefügt und das Gemisch während 2 Stunden auf 50 °C erwärmt. Danach wurde durch Zugabe von festem Natriumhydrogencarbonat neutralisiert und anschließend filtriert; das Filtrat wurde eingedampft und der Rückstand an Kieselgel mit Toluol/Essigester (2:1) chromatographiert. Man erhielt auf diese Weise 375 mg (90%) 2R,4'R,8'R-α-Tocopherol (d-α-Tocopherol) als schwach gelbliches Öl. Für die optische Reinheit des auf obige Weise erhaltenen d-α-Tocopherols wurde ein Wert von 95% bestimmt.

**Patentansprüche für die Vertragsstaaten BE CH DE FR GB IT LI NL**

1. Verfahren zur Herstellung von Hydrochinonderivaten, dadurch gekennzeichnet, daß man eine Verbindung der allgemeinen Formel I

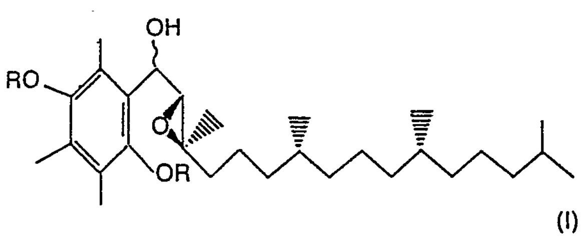

(I)

worin R eine übliche Ätherschutzgruppe darstellt, mittels eines komplexen Hydrides regioselektiv reduziert, daß man, gewünschtenfalls, eine so erhaltene Verbindung der allgemeinen Formel II

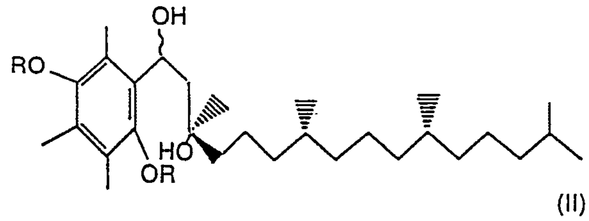

(II)

worin R die obige Bedeutung hat, in an sich bekannter Weise in eine Verbindung der allgemeinen Formel III

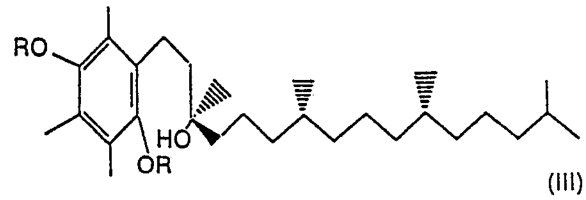

(III)

worin R die obige Bedeutung hat, überführt.

2. Verfahren zur Herstellung von d-α-Tocopherol, dadurch gekennzeichnet, daß man eine Verbindung der allgemeinen Formel I

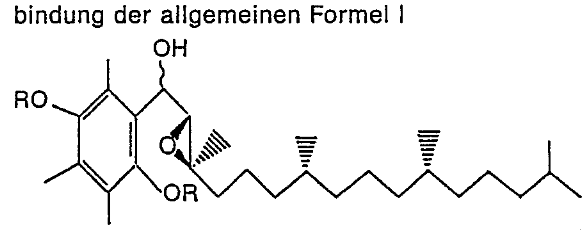

(I)

worin R eine übliche Ätherschutzgruppe darstellt, mittels eines komplexen Hydrides regioselektiv reduziert, daß man eine so erhaltene Verbindung der allgemeinen Formel II

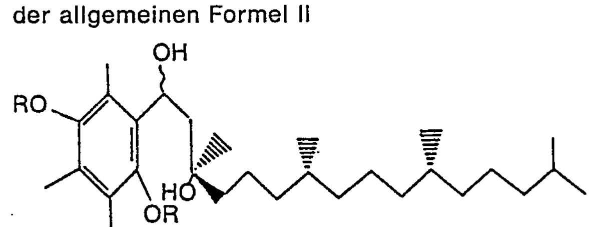

(II)

worin R die obige Bedeutung hat, in an sich bekannter Weise in eine Verbindung der allgemeinen Formel III

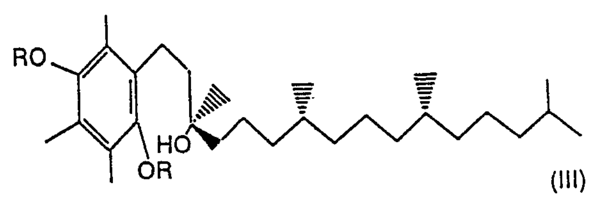

(III)

worin R die obige Bedeutung hat, überführt und diese in bekannter Weise in d-α-Tocopherol der Formel IV

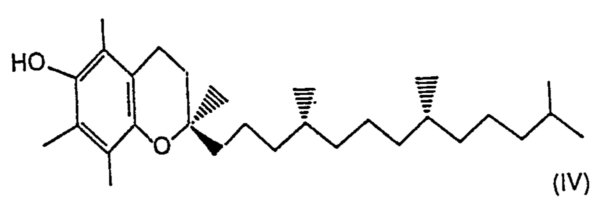

(IV)

überführt.

3. Verfahren nach Anspruch 1 oder 2, dadurch gekennzeichnet, daß man die regioselektive Reduktion mittels Natrium bis(2-methoxy-äthoxy)aluminiumhydrid, Lithiumaluminiumhydrid, Lithiumborhydrid, Aluminiumborhydrid, Aluminiumhydrid oder Diboran durchführt.

4. Verfahren nach Anspruch 1, 2 oder 3, dadurch gekennzeichnet, daß man die regioselektive Reduktion mittels Natrium bis(2-methoxy-äthoxy)aluminiumhydrid oder Lithiumaluminiumhydrid durchführt.

5. Verbindungen der allgemeinen Formel I

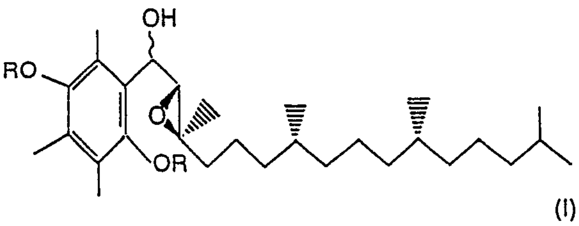

(I)

worin R eine übliche Ätherschutzgruppe darstellt.

6. Die Verbindung der Formel V

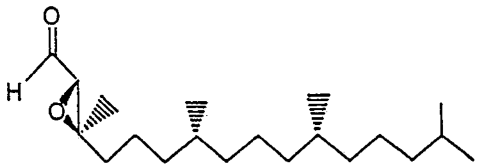

(V)

**Patentansprüche für den Vertragsstaat AT**

1. Verfahren zur Herstellung von Hydrochinonderivaten, dadurch gekennzeichnet, daß man eine Verbindung der allgemeinen Formel I

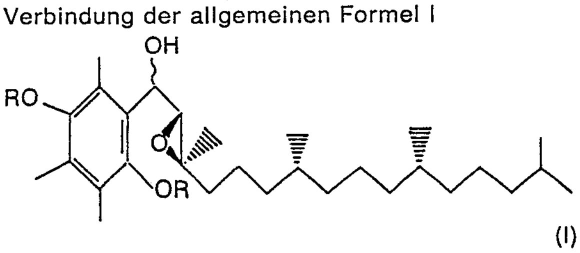

(I)

worin R eine übliche Ätherschutzgruppe darstellt, mittels eines komplexen Hydrides regioselektiv reduziert, daß man, gewünschtenfalls, eine so erhaltene Verbindung der allgemeinen Formel II

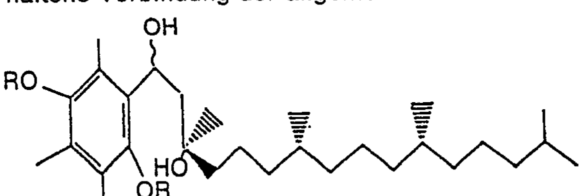

(II)

worin R die obige Bedeutung hat, in an sich bekannter Weise in eine Verbindung der allgemeinen Formel III

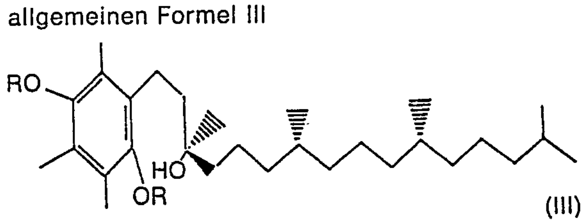

(III)

worin R die obige Bedeutung hat, überführt.

2. Verfahren zur Herstellung von d-α-Tocopherol, dadurch gekennzeichnet, daß man eine Verbindung der allgemeinen Formel I

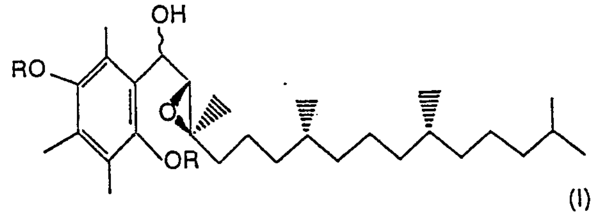

(I)

worin R eine übliche Ätherschutzgruppe darstellt, mittels eines komplexen Hydrides regioselektiv reduziert, daß man eine so erhaltene Verbindung der allgemeinen Formel II

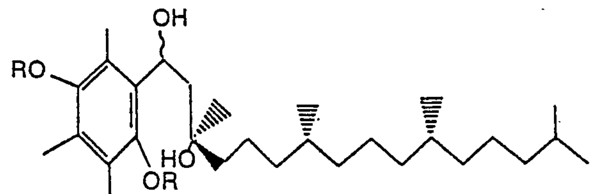

(II)

worin R die obige Bedeutung hat, in an sich bekannter Weise in eine Verbindung der allgemeinen Formel III

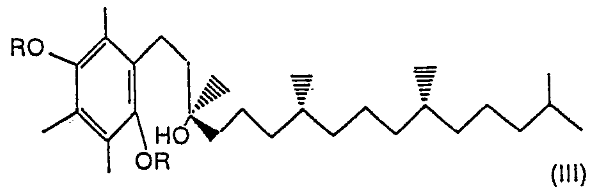

(III)

worin R die obige Bedeutung hat, überführt und diese in bekannter Weise in d-α-Tocopherol der Formel IV

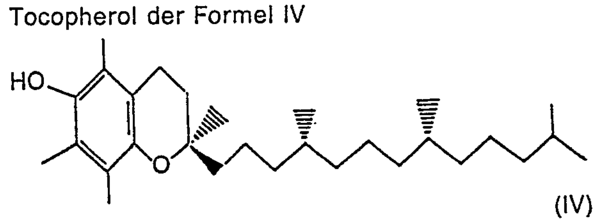

(IV)

überführt.

3. Verfahren nach Anspruch 1 oder 2, dadurch gekennzeichnet, daß man die regioselektive Reduktion mittels Natrium bis(2-methoxy-äthoxy)aluminiumhydrid, Lithiumaluminiumhydrid, Lithiumborhydrid, Aluminiumborhydrid, Aluminiumhydrid oder Diboran durchführt.

4. Verfahren nach Anspruch 1, 2 oder 3, dadurch gekennzeichnet, daß man die regioselektive Reduktion mittels Natrium bis(2-methoxy-äthoxy)aluminiumhydrid oder Lithiumaluminiumhydrid durchführt.

**Claims for the Contracting States BE CH DE FR GB IT LI NL**

1. A process for the manufacture of hydroquinone derivatives, characterized by regioselectively reducing a compound of the general formula I

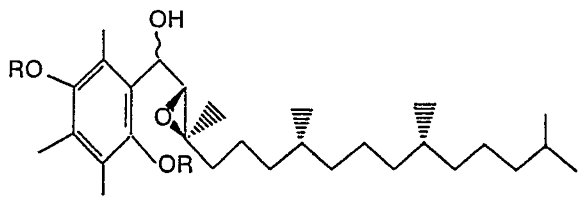

(I)

wherein R represents a conventional ether protecting group,
by means of a complex hydride, if desired converting a thus-obtained compound of the general formula II

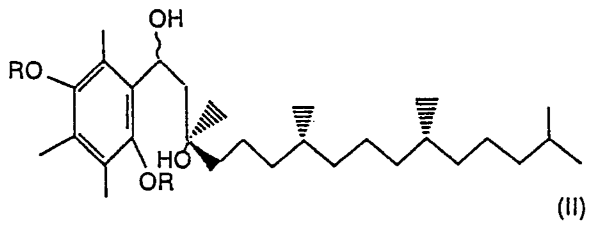

(II)

wherein R has the above significance,
in a manner known per se into a compound of the general formula III

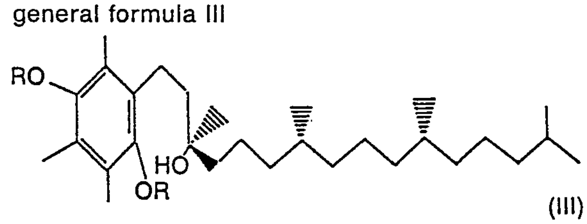

(III)

wherein R has the above significance.

2. A process for the manufacture of d-α-tocopherol, characterized by regioselectively reducing a compound of the general formula I

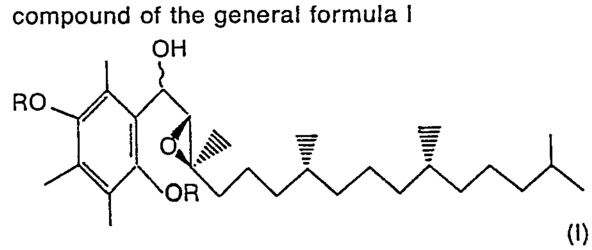

(I)

wherein R represents a conventional ether protecting group,
by means of a complex hydride, converting a thus-obtained compound of the general formula II

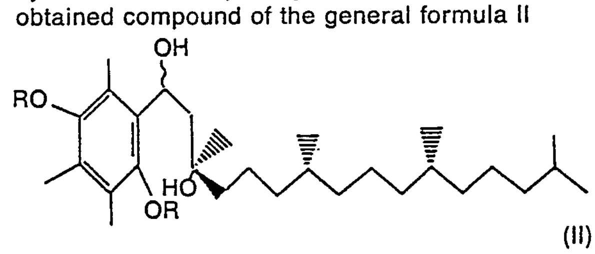

(II)

wherein R has the above significance,
in a manner known per se into a compound of the general formula III

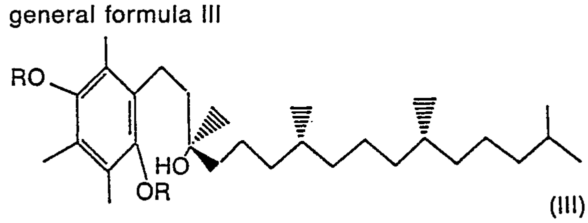

(III)

wherein R has the above significance,
and converting this in a known manner into d-α-tocopherol of the formula IV

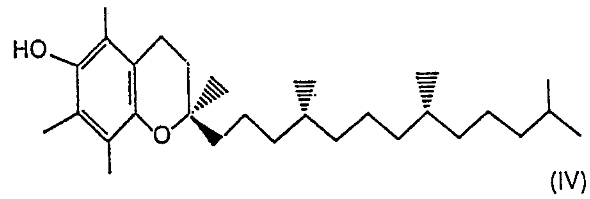

(IV)

3. A process according to claim 1 or 2, characterized in that the regioselective reduction is carried out by means of sodium bis(2-methoxyethoxy)-aluminium hydride, lithium aluminium hydride, lithium borohydride, aluminium borohydride, aluminium hydride or diborane.

4. A process according to claim 1, 2 or 3, characterized in that the regioselective reduction is carried out by means of sodium bis(2-methoxyethoxy)-aluminium hydride or lithium aluminium hydride.

5. Compounds of the general formula I

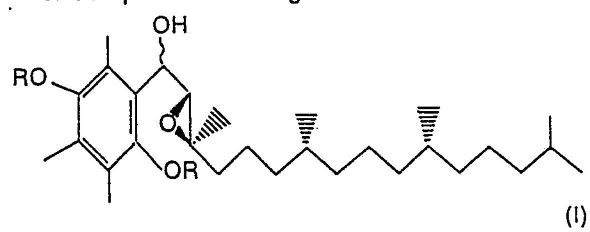

(I)

wherein R represents a conventional ether protecting group.

6. The compound of the formula V

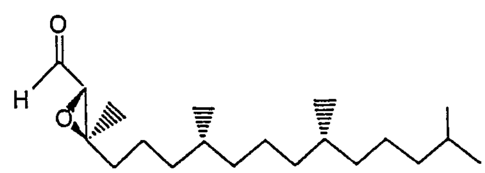

(V)

**Claims for the Contracting State AT**

1. A process for the manufacture of hydroquinone derivatives, characterized by regioselectively reducing a compound of the general formula I

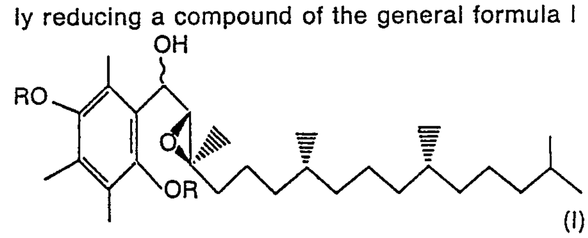

(I)

wherein R represents a conventional ether protecting group,
by means of a complex hydride, if desired converting a thus-obtained compound of the general formula II

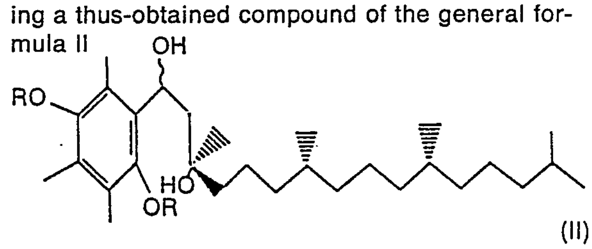

(II)

wherein R has the above significance, in a manner known per se into a compound of the general formula III

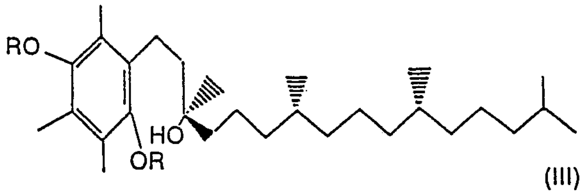

(III)

wherein R has the above significance.

2. A process for the manufacture of d-α-tocopherol, characterized by regioselectively reducing a compound of the general formula I

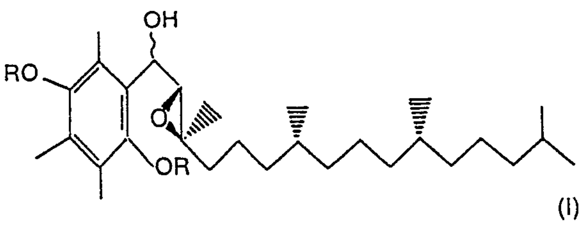

(I)

wherein R represents a conventional ether protecting group, by means of a complex hydride, converting a thus-obtained compound of the general formula II

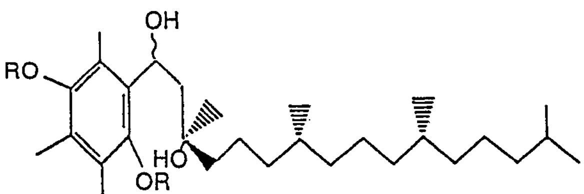

(II)

wherein R has the above significance, in a manner known per se into a compound of the general formula III

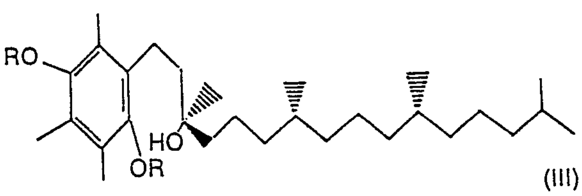

(III)

wherein R has the above significance, and converting this in a known manner into d-α-tocopherol of the formula IV

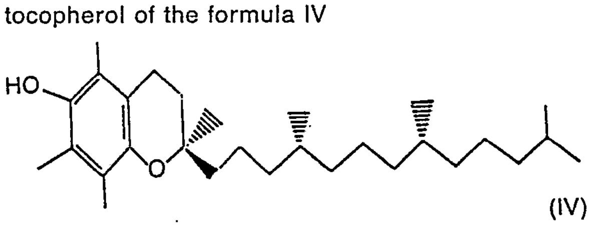

(IV)

3. A process according to claim 1 or 2, characterized in that the regioselective reduction is carried out by means of sodium bis(2-methoxyethoxy)-aluminium hydride, lithium aluminium hydride, lithium borohydride, aluminium borohydride, aluminium hydride or diborane.

4. A process according to claim 1, 2 or 3, characterized in that the regioselective reduction is carried out by means of sodium bis(2-methoxyethoxy)-aluminium hydride or lithium aluminium hydride.

**Revendications pour les Etats Contractants BE CH DE FR GB IT LI NL**

1. Procédé de préparation de dérivés de l'hydroquinone, caractérisé en ce que l'on soumet un composé de formule générale I

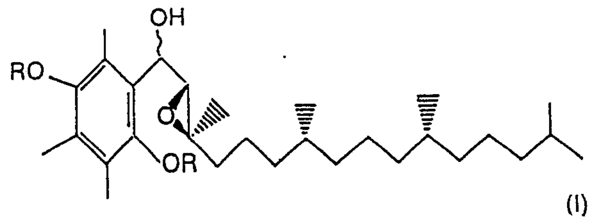

(I)

dans laquelle R représente un groupe protecteur usuel du groupe éther, à une réduction régiosélective à l'aide d'un hydrure complexe, et en ce que si on le désire, on convertit un composé ainsi obtenu, répondant à la formule générale II

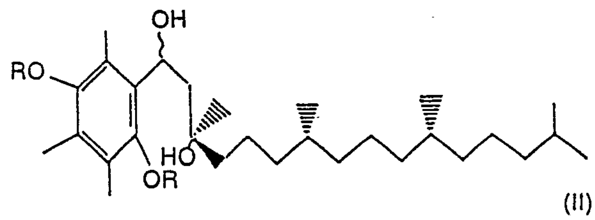

(II)

dans laquelle R a les significations indiquées ci-dessus, de manière connue en soi, en un composé de formule générale III

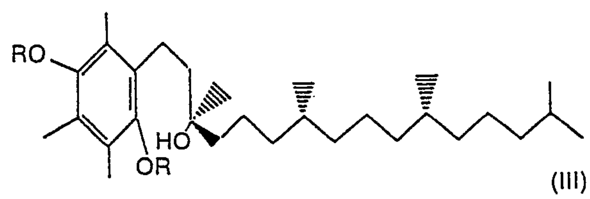

(III)

dans laquelle R a les significations indiquées ci-dessus.

2. Procédé de préparation du d-α-tocophérol, caractérisé en ce que l'on soumet un composé de formule générale I

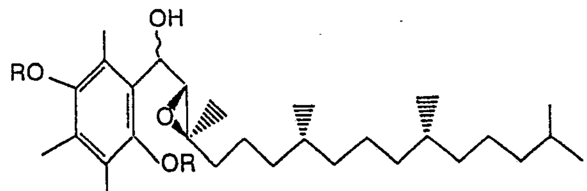

(I)

dans laquelle R représente un groupe protecteur usuel du groupe éther, à une réduction régiosélective à l'aide d'un hydrure complexe, en ce que l'on convertit un composé ainsi obtenu répondant à la formule générale II

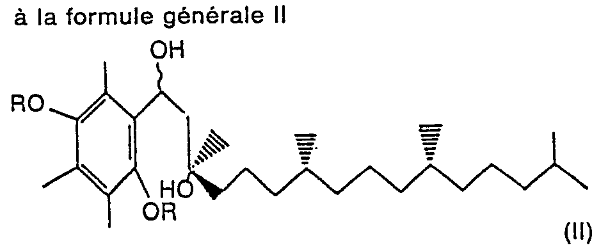

(II)

dans laquelle R a les significations indiquées ci-dessus, de manière connue en soi, en un composé de formule générale III

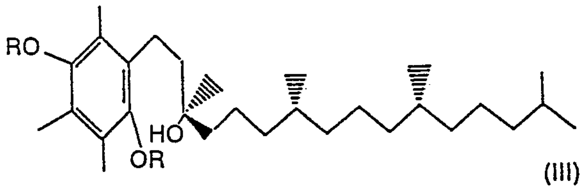

(III)

dans laquelle R a les significations indiquées ci-dessus, qu'on convertit de manière connue en le d-α-tocophérol de formule IV

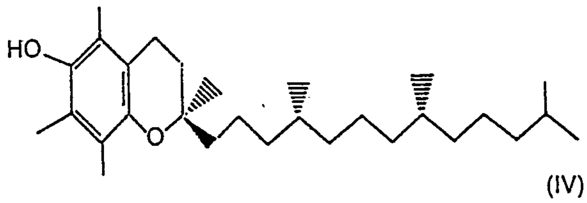

(IV)

3. Procédé selon la revendication 1 ou 2, caractérisé en ce que la réduction régiosélective est effectuée à l'aide de l'hydrure de sodium et de bis-(2-méthoxy-éthoxy)-aluminium, de l'hydrure de lithium et d'aluminium, du borohydrure de lithium, du borohydrure d'aluminium, de l'hydrure d'aluminium ou de diborane.

4. Procédé selon la revendication 1, 2 ou 3, caractérisé en ce que l'on procède à la réduction régiosélective à l'aide de l'hydrure de sodium et de bis-(2-méthoxy-éthoxy)-aluminium ou à l'aide de l'hydrure de lithium et d'aluminium.

5. Composés de formule générale I

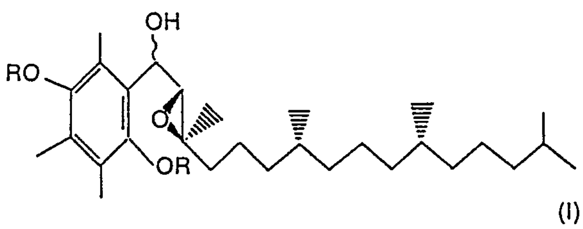

(I)

dans laquelle R représente un groupe protecteur usuel du groupe éther.

6. Le composé de formule V

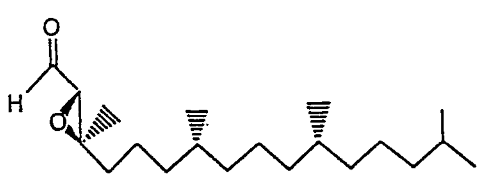

(V)

**Revendications pour l'Etat contractant AT**

1. Procédé de préparation de dérivés de l'hydroquinone, caractérisé en ce que l'on soumet un composé de formule générale I

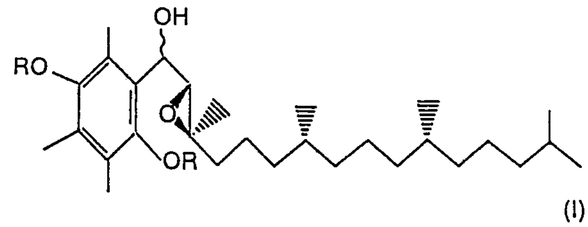

(I)

dans laquelle R représente un groupe protecteur usuel du groupe éther, à une réduction régiosélective à l'aide d'un hydrure complexe, et en ce que si on le désire, on convertit un composé ainsi obtenu, répondant à la formule générale II

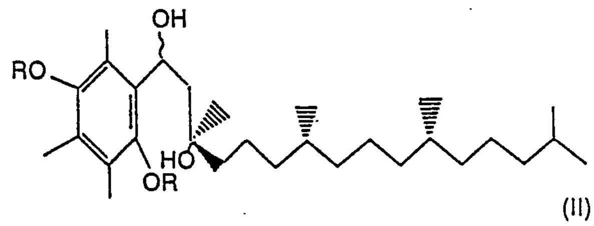

(II)

dans laquelle R a les significations indiquées ci-dessus, de manière connue en soi, en un composé de formule générale III

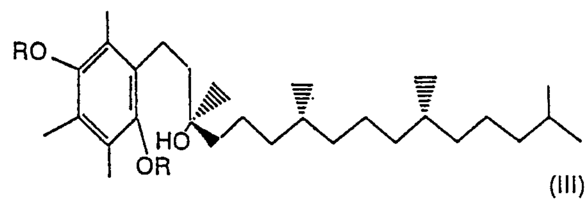

(III)

dans laquelle R a les significations indiquées ci-dessus.

2. Procédé de préparation du d-α-tocophérol, caractérisé en ce que l'on soumet un composé de formule générale I

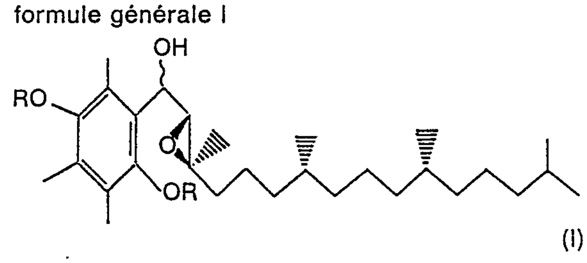

(I)

dans laquelle R représente un groupe protecteur usuel du groupe éther, à une réduction régiosélective à l'aide d'un hydrure complexe, en ce que l'on convertit un composé ainsi obtenu répondant à la formule générale II

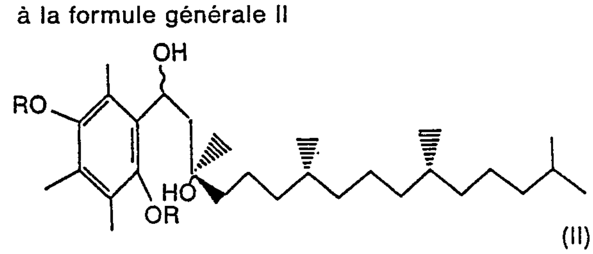

(II)

dans laquelle R a les significations indiquées ci-dessus, de manière connue en soi, en un composé de formule générale III

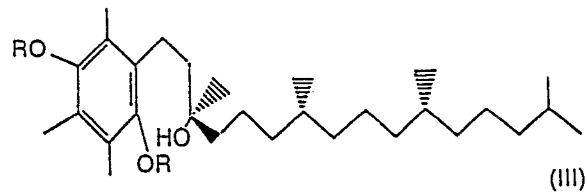

(III)

dans laquelle R a les significations indiquées ci-dessus, qu'on convertit de manière connue en le d-α-tocophérol de formule IV

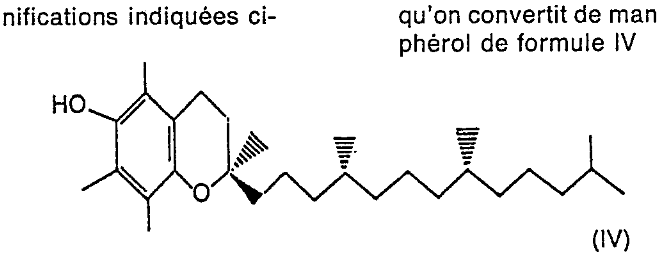

(IV)

3. Procédé selon la revendication 1 ou 2, caractérisé en ce que la réduction régiosélective est effectuée à l'aide de l'hydrure de sodium et de bis-(2-méthoxy-éthoxy)-aluminium, de l'hydrure de lithium et d'aluminium, du borohydrure de lithium, du borohydrure d'aluminium, de l'hydrure d'aluminium ou de diborane.

4. Procédé selon la revendication 1, 2 ou 3, caractérisé en ce que l'on procède à la réduction régiosélective à l'aide de l'hydrure de sodium et de bis-(2-méthoxy-éthoxy)-aluminium ou à l'aide de l'hydrure de lithium et d'aluminium.